(19) Europäisches Patentamt
European Patent Office
Office européen des brevets

(11) EP 4 759 818 A1

(12) **DEMANDE DE BREVET EUROPEEN**

(43) Date de publication:
**17.06.2026 Bulletin 2026/25**

(21) Numéro de dépôt: **25218621.8**

(22) Date de dépôt: **26.11.2025**

(51) Classification Internationale des Brevets (IPC):
***C07F 7/18*** *(2006.01)* ***A61K 8/58*** *(2006.01)*
***A61Q 19/00*** *(2006.01)* ***A61Q 19/08*** *(2006.01)*
***C07D 247/02*** *(2006.01)*

(52) Classification Coopérative des Brevets (CPC):
**C07F 7/1804; A61K 8/585; A61Q 19/00; A61Q 19/08**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **13.12.2024 FR 2414124**

(71) Demandeur: **Exsymol**
**98000 Monaco (MC)**

(72) Inventeur: **CAPPONI, Marco**
**18100 IMPERIA (IT)**

(74) Mandataire: **Murgitroyd & Company**
**165-169 Scotland Street**
**Glasgow G5 8PL (GB)**

(54) **COMPLEXES DE SILICIUM ORGANIQUE ET LEURS APPLICATIONS**

(57) La présente invention concerne de nouveaux complexes de silicium organique, ainsi que leurs applications.



Processed by Luminess, 75001 PARIS (FR)

## Description

### Domaine de l'invention

La présente invention concerne de nouveaux complexes de silicium organique, ainsi que leurs applications.

### Etat de la technique

Partout dans le monde, les gens vivent plus longtemps. D'ici à 2030, une personne sur six dans le monde aura 60 ans ou plus (Bulletin de l'OMS « Vieillissement et santé », 1er Oct. 2024). Un des enjeux majeurs de ce XXIe siècle reste donc la santé et la lutte contre le vieillissement, que certains définissent, du moins d'un point de vue physiologique, comme le produit de l'accumulation au fil du temps d'un vaste éventail de dommages moléculaires et cellulaires, et qui altère en fin de compte les capacités physiques et intellectuelles de l'organisme (Boismal F. et al., Médecine/Sciences, 2020, vol.36, pp.1163-72). Or, parmi la multitude d'études initiées depuis des décennies par la communauté scientifique (gérontologues, biologistes moléculaires, immunologistes, neuroscientifiques, etc.) pour mieux comprendre les mécanismes du vieillissement chronologique du corps humain et développer des moyens de ralentir ou de prévenir ses effets négatifs, il est bien acquis en physiopathologie que l'un des biomarqueurs de cette sénescence générale est la mesure du vieillissement moléculaire des protéines (Jaisson S. et al., Médecine/Sciences, 2017, vol.33, pp.176-182). En effet, tout au long de leur séjour dans les cellules vivantes de l'organisme, cette grande classe de macromolécules biologiques est exposée, de manière cumulative, à des réactions délétères qui contribuent progressivement à l'altération de leurs propriétés structurales et fonctionnelles, affectant dès lors la régulation de leurs interactions moléculaires et cellulaires. D'un point de vue mécanistique, il est également admis que cet ensemble réactionnel délétère fait intervenir, avec le temps, divers processus complexes, largement réunis dans la littérature sous le vocable de "Modifications Post-Traductionnelles Non Enzymatiques - MPTNE)" (acronyme "NEPTMs" en anglais) (Jaisson S. et al., Adv. Clin. Chem., 2018, vol.84, pp.1-38). En substance et en termes simples, il s'agit de réactions chimiques, non programmées mais inéluctables, qui se caractérisent par une fixation non régulée, souvent irréversible, de métabolites simples sur les résidus (groupements fonctionnels) d'acides aminés des protéines. Les plus étudiées parmi les susmentionnées MPTNEs affectant les protéines sont au nombre de quatre (Gillery P. et al., Clin. Chem. Lab. Med., 2013, vol.92, pp.228-238). Il y a tout d'abord la réaction d'oxydation, avec une nuisance apportée par l'action d'espèces réactives de l'oxygène (acronyme "ROS" en anglais), notamment des radicaux libres oxygénés, et la formation résultante de métabolites réactifs sur les protéines. Il y a aussi les réactions non enzymatiques dites de carbonylation et de carbamylation, qui correspondent respectivement à la fixation de dérivés/espèces carbonylé(e)s réactif(ve)s (acronyme "RCC" ou "RCS" en anglais) et d'acide isocyanique (source : urée) sur les groupements aminés de ces mêmes protéines. Enfin figurent les réactions, très étudiées, dites de glycosylation et de glycoxydation non enzymatiques des protéines, les secondes correspondant aux premières mais associées et accentuées en présence d'un environnement oxydant. Elles consistent en une condensation de sucres réducteurs, tel le glucose ou le fructose, sur les fonctions aminées N-terminales de certains constituants protéiques, lipoprotéiques ou encore nucléoprotéiques. Impactées de la sorte, les biomolécules correspondantes (protéines, lipides et acides nucléiques) réticulent puis se rigidifient de manière irréversible avec l'accumulation de liaisons croisées covalentes inter-fibres, à l'image de changements observés sur des fibres de collagène glycosylées par un excès de glucose lors de la mise au point *in vitro* d'un modèle expérimental destiné au suivi du diabète sucré (Kent M.J.C., Biochem. J., 1985, vol.225, pp.745-752). Corrélé d'ailleurs à l'hyperglycémie sanguine, il est souligné qu'une telle glycosylation non enzymatique des protéines, et ses répercussions, n'ont pas qu'une origine endogène dans l'organisme. Le phénomène est susceptible en effet d'être amplifié par un apport exogène, par exemple celui de l'alimentation, précisément à travers la coloration et le brunissement excessifs des protéines que notre cuisine moderne tend à donner aux denrées alimentaires sous l'effet de fortes cuissons. Dès lors, il en résulte une "caramélisation des protéines" (Defaye J. et al., L'Actualité Chimique, 2000, pp.24-27) qui n'est rien d'autre qu'une MPTNE décrite supra et qui est susceptible d'engendrer après ingestion, lorsque la dégradation ou l'excrétion des molécules néoformées à la cuisson est insuffisante, une pollution/nuisance alimentaire (cancérogénicité, activités biologiques pro-oxydante et inflammatoire) impliquée dans la physiopathologie du vieillissement de l'organisme et l'amplification des complications des diabètes (Schlienger J.L., Médecine des Maladies Métaboliques, 2022, vol.6, pp.567-572). Par ailleurs dans le processus général de genèse des MPTNEs, il survient ultimement (en phase tardive après la fixation des métabolites sur les groupements fonctionnels d'acides aminés des protéines) une libération, couplée à leur accumulation tissulaire, d'un ensemble hétérogène, chimiquement diversifié, de composés regroupés sous cet autre vocable scientifique de "Produits dérivés des modifications post-traductionnelles non enzymatiques" (acronyme "PTMDP" en anglais pour "Post-Translational Modification Derived Products"). En bref, tous ces produits ainsi libérés et accumulés témoignent du degré de modification et de vieillissement moléculaire des protéines, et donc, par répercussion, des vieillissements cellulaire et tissulaire de l'organisme. Parmi les principaux PTMDPs générés lors des susdites MPTNEs, les plus couramment décrits et commentés sont la méthionine sulfoxyde, la dityrosine et la 3-nitrotyrosine pour la réaction d'oxydation, la 4-hydroxy-2-nonénal-lysine, la malondialdéhyde-lysine, la $N^{\varepsilon}$-carboxyéthyl-lysine pour la réaction de carbonylation, l'homocitrulline pour la réaction de carbamylation (Jaisson S. et al., Médecine/Sciences, 2017, vol.33, pp.176-182 et références citées ; Gillery P. et al., Clin. Chem. Lab. Med., 2013, vol.92, pp.228-238 et références citées). Pour les réactions de glycosylation et de glycoxydation non enzymatiques des protéines, ces PTMDPs peuvent être, après une suite de réactions en chaîne et des réarrangements moléculaires complexes (formation d'une base de Schiff instable puis réarrangement moléculaire dit d'Amadori), la pentosidine, des $\alpha$-cétoaldéhydes tels que le glyoxal, le méthylglyoxal ou encore le 3-déoxyglucosone (Gillery P. et al., Clin. Chem. Lab. Med., 2013, vol.92, pp.228-238 et références citées ; Tessier F.J., Pathologie Biologie, 2010, vol.58, pp.214-219 ; ). Certains de ces PTMDPs générés lors des susdites MPTNEs constituent certes des biomarqueurs d'intérêt utilisés en biologie médicale et du vieillissement, et ce en permettant le suivi expérimental d'un processus physiologique ou de maladies métaboliques liées à l'âge (Gillery P., J. Med. Biochem., 2011, vol.30, ppp.201-206). Cependant au titre d'une autre connaissance admise, et non des moindres, d'autres PTMDPs, en particulier ceux issus de la fixation spontanée de sucres réducteurs aux protéines, sont considérés comme de véritables toxines endogènes aux niveaux moléculaire et cellulaire (Gillery P. et al., Clin. Chem. Lab. Med., 2014, vol.52, pp.33-38), au point même que le terme "glycotoxines" s'affiche aussi dans la littérature pour désigner, de manière plus globale, l'ensemble des produits et sous-produits délétères résultant dudit processus de fixation de sucres réducteurs aux protéines (Monteiro-Alfredo T. et al., Diabetology, 2022, vol.3, pp.596-605). A titre informatif, il en va d'ailleurs de la sorte par exemple, et dans un article au titre préoccupant, bien qu'interrogatif, avec « *Glycotoxins : a possible threat to health* ? » (Odetti P. et al., Mediterr. J. Nutr. Metab., 2008, vol.1, pp. 63-67), et dans un autre article plus récent qui aborde la cytotoxicité affichée par certaines de ces glycotoxines une fois celles-ci prisonnières des cellules (Kuzan A., Biomed. Rep., 2021, vol.14, pp.1-8). Il en sera d'ailleurs d'un même emploi lexical dans la suite de l'exposé ci-après. En tout état de cause, l'accumulation de ces entités "PTMDPs" au cours du vieillissement de l'organisme a été clairement démontrée, en particulier au niveau de la peau et des vaisseaux (Jaisson S. et al., Médecine/Sciences, 2017, vol.33, pp.176-182 et références citées). La présente invention concerne justement le tissu cutané à travers la recherche de moyens pour s'opposer à de telles glycotoxines et leurs actions dommageables résultantes, avec l'objectif *in fine* de contrecarrer le processus de vieillissement (moléculaire) affectant une typologie singulière de protéines cutanées, celles des protéines dites "à demi-vie longue", c'est-à-dire celles dont les demi-vies peuvent atteindre plusieurs dizaines d'années et constituer de ce fait des cibles privilégiées pour des réactions aussi cumulatives et irréversibles que les MPTNEs (Jaisson S. et al., Médecine/Sciences, 2017, vol.33, pp.176-182). Trois protéines à demi-vie longue caractéristiques de la peau sont particulièrement affectées par le phénomène : le collagène, l'élastine et la fibronectine (Buszka A., Aesthetic Cosmetology and Medicine, 2023, vol.12, pp.23-278). En effet, alors que les deux premières sont essentielles au maintien de la structure et de l'élasticité de la peau et que la troisième assure un rôle clef dans l'organisation du derme et de sa matrice extracellulaire, les sus-évoquées glycotoxines affichent négativement à leur encontre une capacité à produire et à intensifier le processus, pontant, de liaisons croisées intramoléculaires et intermoléculaires (transversales) pour ces protéines. Il en résulte, à l'instar du collagène, protéine structurelle et de soutien de la peau, une résistance à la dégradation par les enzymes protéolytiques couramment dénommées "MMP" (métalloprotéases matricielles) et qui constituent une entrave au remplacement de fibres protéiques vieillies par des fibres nouvellement synthétisées, conduisant ainsi en définitive à des fibrilles protéiques qui s'épaississent. Au-delà de ces aspects, un ralentissement du processus de cicatrisation des plaies de la peau, une rigidification et réduction de l'élasticité du tissu cutané, ou encore une visibilité accrue des rides, sont également rapportés dans le susdit article "Buszka A." (Gkogkolou P. et al., Dermato-Endocrinology, 2012, vol.4, pp.259-270). En matière de stratégies destinées à lutter dans l'organisme contre la présence et la nocuité de telles glycotoxines endogènes et/ou apportées par notre alimentation moderne occidentale, quatre concepts d'action dite "générale" ont tout récemment été signalées (Wang L. et al., Exp. Derm., 2024, vol.33, e15065). Tout d'abord, l'on trouve logiquement, couplée ou non avec celle d'un contrôle sévère de la glycémie, l'option d'un régime alimentaire pauvre en aliments sucrés et en produits de cuisson trop brunis (grillés) puisque ces derniers sont l'un des facteurs déterminants de la production de glycotoxines (J. Uribarri et al., J. Am. Diet. Asso., 2010, vol.110, pp.911-916). Il y a ensuite l'option d'une action à visée antioxydante, préventive, cherchant à réduire les processus oxydatifs. En effet, la connaissance des réactions biochimiques affectant la nature des protéines par l'accrochage d'un ou plusieurs sucres démontre qu'elles s'accompagnent généralement de réactions d'oxydation et que, au titre des MPTNEs susmentionnées, les altérations structurales que toutes deux engendrent sur les protéines s'en trouvent potentialisées (Gillery P., J. Soc. Biol., 2001, vol.195, pp.387-390). Les substances de référence pour cette typologie d'agents antioxydants sont notamment la N-acétyl-cystéine, les vitamines C et E, la quercétine (Wang L. et al., Exp. Derm., 2024, vol.33, e15065). Enfin et surtout, il y a l'action d'agents multiples, purement synthétiques ou d'origine naturelle, réunis et réduits parfois à leur aptitude d'agent inhibiteur, capables en tout cas d'interagir sur cette condensation non régulée de sucres aux protéines, et ce à des étapes différentes (précoce ou tardive) de ladite glycosylation et selon divers modes d'action chimique que certains auteurs (Zheng W. et al., Nutrients, 2022, vol.14, pp.1-19) ont compilé en sous-catégories: "pre-Amadori inhibitors", "post-Amadori inhibitors", "crosslinking breakers", etc. (à comprendre pour ces derniers des entités capables de briser la réticulation protéique déjà installée). Quelques molécules archétypes pour ces agents inhibiteurs sont, pêle-mêle, le pyridoxal-5'-phosphate, l'aspirine, l'aminoguanidine, la pyridoxamine, la benfotiamine, le beta-alanyl-histidine, le chlorure de 4,5-diméthyl-3-phénacylthiazolium (alagebrium) (Uceda A.B. et al., Biophys. Rev., 2024, vol.16, pp.189-218 et références citées) ou encore l'acide rosmarinique, composé phénolique naturel présent notamment dans le romarin (Velichkova S. et al., Planta Med., 2021, vol.87, pp.780-802). En tout état de cause pour la peau et malgré cette mise à disposition, multiple et variée, de solutions destinées à prévenir/lutter contre la présence/action de tels PTMDPs et autres glycotoxines, il est relevé, dans cet ensemble de connaissances générales, un facteur limitant, et potentiellement de fait un « challenge » cosmétique d'intérêt pour les industriels. En effet, à l'image du très récent article "Wang L. et al." susmentionné et de sa conclusion, il est rapporté que les études menées jusqu'à ce jour pour évaluer l'effet sur la peau de ces substances existantes ne rendent compte finalement que d'indices et d'une action modérée - qualifiée en anglais de "usually modest" - dans la réduction des dommages engendrés par la présence cutanée de ces PTMDPs et autres glycotoxines, et qu'il en découle aujourd'hui un véritable besoin - étiquetée même en anglais de "urgent need" dans le susdit article "Wang L. et al." - de développer des agents toujours plus efficaces/performants vis-à-vis d'une telle problématique cutanée. Le constat est d'ailleurs partagé dans l'autre très récent article susmentionné "Uceda A.B. et al." à travers les perspectives affichées en fin d'article où il y est souligné, de manière plus générale, que l'efficacité clinique mais aussi le profil d'innocuité de ces substances existantes ne sont pas toujours pleinement établis. Le comportement sur la peau de certaines de ces substances existantes, l'aminoguanidine par exemple, inhibiteur de référence vis-à-vis des "RCCs" évoqués plus haut, d'intérêt pour le piégeage d'intermédiaires carbonylés, affiche défavorablement des effets secondaires indésirables, notamment, dans le cas de l'aminoguadinine susnommée, une élévation des phénomènes oxydatifs et une éruption cutanée de type lupus (Song Q. et al., Biomed. Pharmacotherap., 2021, vol.140, 111750). Ainsi, la présente invention s'inscrit sans réserve dans un même défi et besoin de mettre à disposition [sur le marché des actifs cosmétiques et/ou dermocosmétiques d'intérêt pour la prévention et la lutte contre le vieillissement moléculaire affectant les protéines cutanées et ses conséquences physiologiques : modification de structure, de fonction et d'antigénicité des protéines, modification des interactions entre protéines et cellules, etc. ] un agent qui puisse constituer une solution alternative aux ingrédients actuels de l'état de la technique, avec cependant des performances biologiques améliorées/accrues/bonifiées par rapport à ces derniers et la garantie conjuguée d'une innocuité à l'attention de l'utilisateur. Plus précisément, les objectifs recherchés par la demanderesse ont consisté à concevoir et développer une nouvelle entité chimique originale, dotée d'un certain nombre d'effets techniques avantageux, sélectionnés parmi, et avant toute chose, une efficacité démontrée et puissante sur une ou plusieurs problématiques données autour du vieillissement moléculaire des protéines à renouvellement lent de la matrice extra-cellulaire de la peau, une biodisponibilité cutanée élevée, une stabilité tant dans le temps que dans la formulation du produit fini, mais aussi une toxicologie et sécurité d'utilisation établies.

**Exposé de l'invention**

La présente invention a donc été développée dans le contexte de recherche et cahier des charges tels qu'indiqués supra. La demanderesse a cependant très vite opté, et de manière délibérée, pour une stratégie en chimie moléculaire qui s'écarte d'une primo-approche visant d'emblée à chercher à améliorer - par l'introduction d'une modification structurale (greffage) tel avec l'assistance préalable de la chimie *in silico* ou celle de l'intelligence artificielle (toutes deux prégnantes aujourd'hui pour réaliser du design moléculaire et/ou prédire des propriétés *in vivo)* - le comportement biologique de l'un quelconque, connu, des composés d'intérêt de l'art antérieur vis-à-vis du processus de glycosylation non enzymatique des protéines. Et c'est ainsi que, dans le cadre de cette option jugée plutôt à contre-courant des tendances actuelles, la demanderesse est parvenue, de manière surprenante, à mettre au point un nouveau complexe moléculaire combinant en interaction (chimie supramoléculaire) deux substances - non modifiées -. Précisément, et en particulier dans le respect de ratio et conditions maîtrisés pour l'obtention d'un tel complexe moléculaire, la demanderesse est parvenue à associer, à l'état de complexe stabilisé par des liaisons intermoléculaires faibles, deux familles de composés affichant chacune séparément un historique d'intérêt dans la formulation de produits cosmétiques, exemplifié avec leur présence au dernier glossaire des dénominations communes des ingrédients cosmétiques établi par la Commission européenne à travers la « *Décision d'exécution (UE) 2022/677 du 31 mars 2022 portant modalités d'application du règlement (CE) n° 1223/2009 du Parlement européen et du Conseil »,* ce dernier ayant pour rappel et objet de rendre les produits cosmétiques vendus dans l'Union européenne plus sûrs en établissant des exigences strictes en matière de sécurité pour protéger la santé humaine. En l'espèce, la première de ces familles impliquée dans la formation du susdit complexe moléculaire est une sélection limitée et spécifique de dérivés organo-silanols développés par la demanderesse, de formule générale (I) suivante :

$$(R)_x\text{-}Si(OH)_{4-x} \qquad (I)$$

dans laquelle :

- le radical R est un groupement alkyle en $C_1$-$C_4$, linéaire ou ramifié, de préférence en $C_1$-$C_2$, éventuellement substitué

par au moins un groupement hydroxyle, de préférence R est un groupement méthyle, et

- x est égal à 1 ou 2.

Il convient de souligner que cette sélection spécifique de dérivés organo-silanols portant deux ou trois groupes hydroxyle libres sur l'atome de silicium se distingue clairement, notamment en termes de structure mais également d'activité, des polymères de type siloxane dépourvus de groupes hydroxyles, fréquemment illustrés dans la littérature par le très commun "PDMS" ou PolyDiMethylSiloxane - comme par exemple dans la demande de brevet US2015/0004113 - et qui constituent des matériaux dotés d'une inertie biologique et chimique.

Quant à la seconde de ces familles engagée dans ce nouveau complexe moléculaire, il s'agit d'une sélection limitée et spécifique de composés organiques à motif imidazole de formule générale (II) suivante :

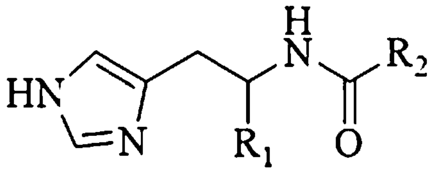

(II)

dans laquelle :

- $R_1$ est -H, $-CO_2H$ ou $-CH_2OH$,
- $R_2$ est :

  i) soit une chaîne alkyle de formule (III) suivante :

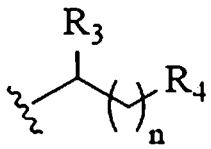

(III)

  dans laquelle :

  - $R_3$ est -H ou $-NH_2$ ;
  - $R_4$ est $-CO_2R_5$ ou $-NHR_5$, avec $R_5$ étant -H ou $-COCH_3$;
  - n est égal à 1 ou 2 ;

  ii) soit un hétérocycle choisi parmi :

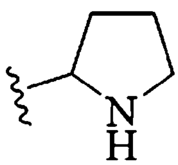

et

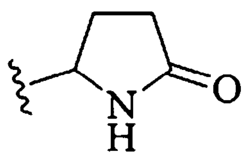

.

L'invention a donc pour premier objet un complexe de silicium organique formé entre :

a) un organo-silanol de formule générale (I) suivante :

$$(R)_x\text{-Si(OH)}_{4-x} \qquad \text{(I)}$$

dans laquelle :

- le radical R est un groupement alkyle en $C_1$-$C_4$, linéaire ou ramifié, de préférence en $C_1$-$C_2$, éventuellement substitué par au moins un groupement hydroxyle, de préférence R est un groupement méthyle, et
- x est égal à 1 ou 2 ; et

b) au moins un composé organique à motif imidazole de formule générale (II) suivante, ou l'un de ses sels cosmétiquement ou dermocosmétiquement acceptables :

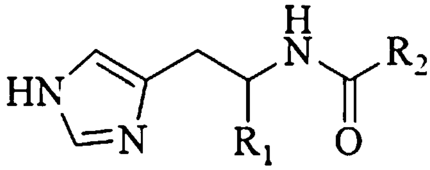

(II)

dans laquelle :

- $R_1$ est -H, $-CO_2H$ ou $-CH_2OH$,
- $R_2$ est :

i) soit une chaîne alkyle de formule (III) suivante :

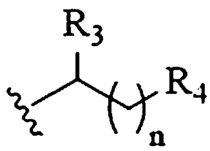

(III)

dans laquelle :

- $R_3$ est -H ou $-NH_2$ ;
- $R_4$ est $-CO_2R_5$ ou $-NHR_5$, avec $R_5$ étant -H ou $-COCH_3$;
- n est égal à 1 ou 2 ;

ii) soit un hétérocycle choisi parmi:

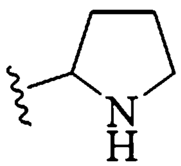
et
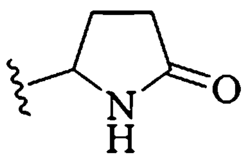

ledit complexe étant formé via l'établissement d'au moins une (par exemple deux ou trois) liaison faible, de préférence d'au moins une (par exemple deux ou trois) liaison hydrogène, entre au moins un (par exemple deux ou trois) groupement hydroxyle dudit organo-silanol de formule générale (I) et ledit au moins un composé organique à motif imidazole de formule générale (II), de préférence au niveau d'au moins un (par exemple de deux ou trois) des atomes d'azote et d'oxygène dudit au moins un composé organique à motif imidazole de formule générale (II);
le ratio molaire entre ledit organo-silanol de formule générale (I) et ledit au moins un composé organique à motif imidazole de formule générale (II) étant compris entre environ 0,1 et environ 1 (par exemple entre 0,1 et 1).

Selon un mode de réalisation, le complexe selon l'invention est formé via l'établissement de liaisons faibles, de préférence de liaisons hydrogène, entre les groupements hydroxyle dudit organo-silanol de formule générale (I) et les atomes d'azote et d'oxygène dudit au moins un composé organique à motif imidazole de formule générale (II).

Sans être lié par la théorie, les composés organiques à motif imidazole de formule générale (II) jouent le rôle

d'agents stabilisants d'organosilanol via l'établissement d'au moins une liaison faible, de préférence d'au moins une liaison hydrogène, avec au moins un groupement hydroxyle dudit organo-silanol de formule générale (I), tel qu'expliqué supra. A ce titre, ils peuvent également être dénommés « agents stabilisants d'organosilanol » (ou, plus simplement, « agents stabilisants »).

Après des recherches poussées , le choix de la demanderesse s'est en effet finalement arrêté sur une telle nouvelle entité chimique et association intermoléculaire, sous forme de "complexe" tel que défini par les règles de nomenclature de l'Union Internationale de Chimie Pure et Appliquée ("UICPA" en français, ou "IUPAC" en anglais pour "International Union of Pure and Applied Chemistry"), en raison du comportement général avantageux et prometteur que ledit complexe selon l'invention a affiché à travers un faisceau d'effets techniques bénéfiques, tout particulièrement la supériorité expérimentale que ledit complexe selon l'invention a affiché en comparaison de chacun de ses éléments constitutifs pris isolément (à comprendre par "non-engagé dans un même état de complexe "), illustrée par :

- sa capacité à limiter très significativement la formation d'une protéine glycosylée produite par la mise en contact d'un composé dicarbonylé fortement réactif (généré à partir d'un dérivé du glucose après un réarrangement moléculaire d'Amadori tel qu'évoqué en page ci-avant), à savoir le glucosone, sur une protéine de référence analytique (stable et dépourvue d'activité enzymatique), à savoir l'albumine sérique bovine plus connue sous l'acronyme usuel en anglais "BSA" [cf. test 1 ci-après] ;
- sa capacité à réduire très significativement la sécrétion d'une enzyme protéolytique de type métalloprotéase matricielle (MMP) défavorable au renouvellement du collagène fibrillaire de la matrice extra-cellulaire de la peau, en l'occurrence la "MMP-1" telle qu'évoquée en page ci-avant, cette dernière secrétée suite à l'induction délétère (stress) du susnommé glucosone sur des cellules fibroblastiques en culture (acronyme "NHDF" en anglais pour "Normal Human Dermal Fibroblasts") [cf. test 2 ci-après] ;
- enfin, sa capacité à stimuler notablement l'expression d'une autre protéine à longue demi-vie telle qu'évoquée en page ci-avant, la fibronectine, protéine de structure au rôle clef dans l'adhésion/la fixation des cellules dermiques à cette même matrice extra-cellulaire de la peau (Jacob M.P., Médecine/Sciences, 2006, vol.22, pp.273-278) [cf. test 3 ci-après].

De surcroît, et il s'agit là d'un des fondements de la présente invention, il est relevé un phénomène de synergie d'action particulièrement inattendu pour un tel complexe associant, par liaisons faibles, le susdit organo-silanol de formule générale (I) et le susdit au moins un composé organique à motif imidazole de formule générale (II). En effet, ledit état de complexe moléculaire, couplé à ses caractéristiques compositionnelles, a permis, d'une part, de s'opposer significativement à la formation de la "BSA-glucosone" (glycotoxine), d'autre part de stimuler l'expression protéique, bénéfique pour la peau, de la fibronectine, et ce, de manière beaucoup plus efficace - effet largement supérieur - que l'organo-silanol de formule générale (I), seul, ou le même composé organique à motif imidazole de formule générale (II), seul.

Il est ainsi légitimement formulé une telle synergie d'action :

- au regard d'un enseignement théorique apporté pour l'emploi d'une telle locution dans le domaine des produits et substances chimiques, en particulier le fait que (sic) *« il y a effet synergique lorsque l'effet combiné de deux produits chimiques est de beaucoup supérieur à la somme des effets de chaque produit pris individuellement. Par exemple : 2 + 2 >> 4 »* [ https://www.cchst.ca/oshanswers/chemicals/synergism.html ] ;
- à la lumière de l'expérimentation et des résultats obtenus dans chacune des études, puisque l'association des deux composants, sous forme de complexe moléculaire, permet d'obtenir un bien meilleur effet que celui obtenu, à des concentrations d'utilisation équivalentes, pour chacun d'entre eux pris individuellement.

Il est en outre légitimement formulé que ce mode d'action spécifique, avec ce phénomène objectivé de synergie d'action, permet de qualifier le comportement biologique du susdit complexe illustré comme "optimal" ou "doué de propriétés optimales" pour la protection des protéines cutanées à demi-vie longue (collagène, élastine et fibronectine) contre leur vieillissement moléculaire, c'est-à-dire toutes les modifications non enzymatiques que celles-ci subissent naturellement et qui conduisent à des altérations de leurs propriétés structurales et fonctionnelles. Il est enfin légitimement formulé la formation et l'existence d'un état de "complexe" (moléculaire), eu égard, d'une part aux définitions de "complexe" et de "liaison hydrogène" établies par l'Union Internationale de Chimie Pure et Appliquée ("UICPA" en français, ou "IUPAC" en anglais), d'autre part et pour les composés organiques à motif imidazole qui ont été sélectionnés, à leurs régions riches en électrons, marquées par la présence de doublets électroniques non-liants sur certains atomes d'azote et d'oxygène, propices justement à l'établissement de telles interactions de type "liaison hydrogène". En revanche, et bien qu'un même état de "complexe" et liaisons faibles aient déjà été revendiqués par la demanderesse pour le dérivé méthylsilanetriol associé à un sucre monomère de type déoxy-aldohexose (brevet FR3038898), alternativement le dérivé méthylsilanetriol associé à des fragments calibrés d'acide hyaluronique de poids moléculaire compris entre 150 et 750 kDa (brevet EP2172186), ou encore le dérivé méthylsilanetriol en coexistence avec un second organo-silanol, tous deux associés, via un mélange de liaisons faibles et covalentes, à un panel d'agents stabilisants (brevet EP4153603) - ces derniers clairement distincts cependant de la présente sélection de composés organiques à motif imidazole de formule générale (II) - les susdits dérivés organo-silanols de formule générale (I) et composés organiques à motif imidazole de formule générale (II) n'ont jamais été combinés pour la conception d'un tel état de "complexe", ni pour l'utilisation de celui-ci, à des fins cosmétiques et/ou dermocosmétiques, vis-à-vis des protéines à longue demi-vie, en particulier la fibronectine. En outre, un quelconque phénomène de synergie d'action, entre les susdits organo-silanols de formule générale (I) et composés organiques à motif imidazole de formule générale (II), à l'état de "complexe" ou non (simple association), n'a jamais été décrit ni même présagé dans l'état de la technique. De plus, conformément aux objectifs attachés au problème technique posé par la présente invention, le susdit complexe selon l'invention présente *de facto,* du fait de l'appartenance de ses éléments constitutifs pris séparément(pour nombre d'entre eux) à la susmentionnée Décision d'exécution (UE) 2022/677, l'intérêt d'une conformité aux réglementations européennes cosmétique et chimique (respectivement les règlements (CE) n° 1223/2009 et n° 1907/2006 du Parlement Européen et du Conseil) visant à garantir la sécurité des consommateurs. Il est d'ailleurs accessible un large ensemble de données d'évaluation toxicologique - certaines lointaines et donc "rassurantes" pour les formulateurs de produits finis - concernant certains des susdits dérivés organo-silanols de formule générale (I) et composés organiques à motif imidazole de formule générale (II).

Selon un mode de réalisation de l'invention, ledit organo-silanol de formule générale (I) est le monométhylsilanetriol ou le diméthylsilanediol, de préférence le monométhylsilanetriol. Selon un mode de réalisation préféré de l'invention, ledit au moins un composé organique à motif imidazole de formule générale (II) est tel que :

- $R_1$ est -H ou -$CO_2H$ ; et
- $R_2$ est une chaîne alkyle de formule (III) dans laquelle :

    - $R_3$ est -H,
    - $R_4$ est -$NHR_5$ avec $R_5$ étant -H ou -$COCH_3$,
    - n est égal à 1.

Selon un mode de réalisation préféré de l'invention, ledit au moins un composé organique à motif imidazole de formule générale (II) est tel que :

- $R_1$ est -H ou -$CO_2H$ ; et
- $R_2$ est une chaîne alkyle de formule (III) dans laquelle :

    - $R_3$ est -H,
    - $R_4$ est -$NHR_5$ avec $R_5$ étant -H,
    - o n est égal à 1.

Selon un mode de réalisation préféré de l'invention, ledit au moins un composé organique à motif imidazole de formule générale (II) est le composé 3-amino-N-[2-(1H-imidazol-5-yl)ethyl]propanamide, le composé 3-acetamido-N-[2-(1H-imidazol-5-yl)ethyl]propanamide, le composé acide (2S)-2-(3-aminopropanoylamino)-3-(1H-imidazol-5-yl)propanoïque, et/ou le composé acide (4S)-4-amino-5-[2-(1*H*-imidazol-5-yl)ethylamino]-5-oxopentanoïque. Avantageusement, il s'agit du composé 3-amino-N-[2-(1H-imidazol-5-yl)ethyl]propanamide. Les sels dudit au moins un composé organique à motif imidazole de formule générale (II), en particulier des composés préférés susmentionnés, peuvent être obtenus par réaction desdits dérivés et composés avec un acide inorganique, préférentiellement l'acide chlorhydrique, ou un acide organique, en particulier ledit sel cosmétiquement ou dermocosmétiquement acceptable selon l'invention est un chlorhydrate.

Dans un mode de réalisation préféré de l'invention, ledit organo-silanol de formule générale (I) et ledit au moins un composé organique à motif imidazole de formule générale (II) sont respectivement le monométhylsilanetriol et le 3-amino-N-[2-(1H-imidazol-5-yl)ethyl]propanamide.

Dans un mode de réalisation préféré de l'invention, le complexe revendiqué est tel que le ratio molaire entre le susdit organo-silanol de formule générale (I) et le susdit au moins un composé organique à motif imidazole de formule générale (II) est compris entre environ 0,15 et environ 0,8 (par exemple entre 0,15 et 0,8), avantageusement entre environ 0,2 et environ 0,6 (par exemple entre 0,2 et 0,6), encore plus avantageusement entre environ 0,3 et environ 0,5 (par exemple entre 0,3 et 0,5).

Dans un mode de réalisation préféré de l'invention, le complexe selon l'invention se présente sous forme liquide, avantageusement sous forme de solution.

Avantageusement, le complexe selon l'invention est tel que :

i) le ratio molaire entre le susdit organo-silanol de formule générale (I) (par exemple le monométhylsilanetriol) et le

susdit au moins un composé organique à motif imidazole de formule générale (II) (par exemple le composé 3-amino-N-[2-(1H-imidazol-5-yl)ethyl]propanamide) est compris entre environ 0,15 et environ 0,8 (par exemple entre 0,15 et 0,8), encore plus avantageusement entre environ 0,2 et environ 0,6 (par exemple entre 0,2 et 0,6) ; de manière particulièrement préférée ledit ratio molaire étant d'environ 0,4 (par exemple 0,4), et/ou

ii) le pH de la composition (sous forme liquide, avantageusement sous forme de solution) comprenant ledit complexe est compris entre environ 3 et environ 8 (par exemple entre 3 et 8), de préférence entre environ 4 et environ 6 (par exemple entre 4 et 6) ;

de manière préférée, ledit complexe et la composition liquide le comprenant (avantageusement sous forme de solution) présentant les caractéristiques i) et ii) tel qu'illustré par son procédé de préparation [cf. test 4 ci-après].

L'invention a également pour objet une composition, de préférence sous forme liquide, avantageusement sous forme de solution, comprenant, ou consistant essentiellement en, au moins un complexe selon l'invention.

L'invention a également trait à une composition cosmétique ou dermocosmétique, caractérisée en ce que ladite composition comprend (ou consiste essentiellement en), en association avec au moins un excipient cosmétiquement ou dermocosmétiquement acceptable, au moins un complexe selon l'invention, de manière préférée ladite composition étant sous une forme adaptée à une administration par voie topique cutanée et ledit au moins un excipient étant en outre physiologiquement acceptable avec la peau. Avantageusement, la quantité dudit complexe selon l'invention dans les compositions susmentionnées est comprise entre 1 % et 10 % en poids par rapport au poids total de ladite composition, de préférence entre 3 % et 7 % en poids par rapport au poids total de ladite composition. A titre indicatif et non limitatif, lorsque les susdites compositions à usage cosmétique ou dermocosmétique sont adaptées à une administration par voie topique cutanée, elles sont susceptibles de se présenter sous toutes les formes normalement utilisées pour une telle voie d'administration. Ainsi et de manière avantageuse, elles peuvent se présenter sous une forme liquide, avantageusement sous forme d'une solution (par exemple sous forme de solution aqueuse ou hydroalcoolique), éventuellement gélifiée, d'une dispersion du type lotion éventuellement biphasée, d'une émulsion huile-dans-eau ou eau-dans-huile, d'une suspension, d'une lotion, d'une crème, d'un gel aqueux ou hydroalcoolique, ou bien encore sous la forme d'une poudre (micronisée), d'une mousse, d'un sérum, d'une pâte, d'une suspension, d'une dispersion ou d'émulsions multiples pouvant être éventuellement des microémulsions ou des nanoémulsions. En terme d'adjuvants présents dans les compositions susvisées et physiologiquement compatibles avec la peau, l'on peut citer un composé choisi parmi les huiles, les cires, les élastomères de silicone, les tensioactifs, les co-tensioactifs,les solubilisants, les co-solubilisants, les épaississants et/ou gélifiants, les humectants, les émollients, les filtres solaires organiques ou inorganiques, les photostabilisants, les conservateurs à l'exception des conservateurs donneurs d'aldéhydes, les colorants, les charges abrasives, les nacres, les agents matifiants, les agents tenseurs, les agents séquestrants, les parfums, etc., et leurs mélanges. A titre indicatif et non limitatif, un tel adjuvant acceptable dans les domaines d'application visés par la présente invention, présent dans la formule dans une teneur de l'ordre de 0,01 à 20% en poids par rapport au poids total de la composition, préparation et autre complément susvisé(e), est notamment cité dans le dictionnaire "International Cosmetic Ingredient Dictionary and Handbook" publié par le "Personnal Care Product Council (PCPC)" de l'Association cosmétique américaine. Enfin, ces mêmes compositions susvisées peuvent comprendre, au moins un ingrédient actif additionnel, l'homme du métier veillant toutefois à ce que ces éventuels actifs additionnels, ainsi que leurs proportions, soient choisis de telle manière que les propriétés avantageuses reconnues à ces compositions susvisées ne soient pas, ou substantiellement pas, altérées par l'adjonction envisagée. De tels actifs additionnels peuvent être choisis, sans que cette liste ne soit limitative, parmi les agents dépigmentants, les agents photoprotecteurs, les agents stimulant la fonction barrière, les agents hydratants ou humectants, les agents desquamants, les agents apaisants, les agents exfoliants, les agents stimulant la prolifération cellulaire tels les fibroblastes et les kératinocytes, les agents déglycosylants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents exfoliants, les agents agissant sur la microcirculation, etc., et leurs mélanges, un tel ingrédient actif additionnel étant présent dans la formule dans une teneur de l'ordre de 0,0001 à 20% en poids, de préférence de 0,01 à 5% en poids, par rapport au poids total de la composition susvisée.

L'invention concerne également l'utilisation cosmétique ou dermocosmétique non-thérapeutique d'un complexe selon l'invention telle que définie supra, ou d'une composition cosmétique ou dermocosmétique telle que définie précédemment, pour maintenir et/ou augmenter l'expression des protéines de la matrice extracellulaire de la peau saine, de préférence ladite utilisation étant une utilisation par voie topique cutanée. Avantageusement, ladite protéine de la matrice extracellulaire de la peau saine est la fibronectine.

Selon un mode de réalisation préféré, l'utilisation cosmétique ou dermocosmétique non-thérapeutique d'un complexe selon l'invention tel que défini supra, ou d'une composition cosmétique ou dermocosmétique selon l'invention telle que définie précédemment, est :

a) pour maintenir et/ou augmenter les propriétés biomécaniques de la peau saine, préférentiellement la fermeté et/ou l'élasticité de la peau saine ;
b) pour prévenir et/ou corriger des signes de vieillissement de la peau saine (en particulier induits par la glycosylation non enzymatique des protéines à demi-vie longue, de préférence celle affectant la fibronectine) ;
c) pour prévenir et/ou corriger une fibrose tissulaire associée au vieillissement cutané ;
d) comme agent de la lutte contre la rigidification des parois des capillaires dermiques ;
e) pour restaurer le réseau vasculaire cutané altéré avec l'âge ; et/ou
f) comme agent inhibiteur sur la surproduction de la métalloprotéinase matricielle MMP-1 (induite par la glycosylation non enzymatique des protéines à demi-vie longue, de préférence celle affectant le collagène).

Tel qu'indiqué précédemment, l'utilisation cosmétique ou dermocosmétique non-thérapeutique susmentionnée vise la peau « saine » - ou la/les zone(s) de peau saine - par opposition à une peau « malade » - ou une/des zone(s) de peau malade - présentant une pathologie cutanée.
Dans le cas d'une administration topique cutanée, cette utilisation s'effectue sur une ou plusieurs zone(s) de peau « saine(s) », c'est-à-dire une ou plusieurs zone(s) de peau non affectée(s) par une pathologie cutanée.
Selon un mode de réalisation particulier, l'utilisation cosmétique ou dermocosmétique non-thérapeutique susmentionnée s'effectue chez un individu ne présentant pas de pathologie cutanée. Cet individu est qualifié, dans le contexte de la présente invention, de « sujet sain », par opposition à un « sujet malade » présentant une pathologie cutanée.
Un autre objet de l'invention concerne l'utilisation d'au moins un composé organique à motif imidazole de formule générale (II) suivante, ou l'un de ses sels cosmétiquement ou dermocosmétiquement acceptables :

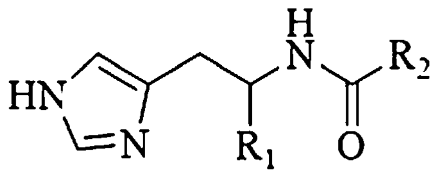

(II)

dans laquelle :

- $R_1$ est -H, $-CO_2H$ ou $-CH_2OH$,
- $R_2$ est :

i) soit une chaîne alkyle de formule (III) suivante :

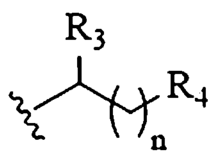

(III)

dans laquelle :

- $R_3$ est -H ou $-NH_2$ ;
- $R_4$ est $-CO_2R_5$ ou $-NHR_5$, avec $R_5$ étant -H ou $-COCH_3$;
- n est égal à 1 ou 2 ;

ii) soit un hétérocycle choisi parmi:

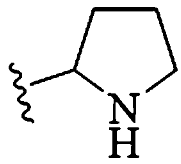 et 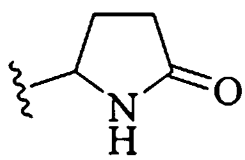

pour stabiliser un organo-silanol de formule générale (I) suivante :

$$(R)_x\text{-}Si(OH)_{4-x} \qquad (I)$$

dans laquelle :

- le radical R est un groupement alkyle en $C_1$-$C_4$, linéaire ou ramifié, de préférence en $C_1$-$C_2$, éventuellement substitué par au moins un groupement hydroxyle, de préférence R est un groupement méthyle, et
- x est égal à 1 ou 2.

Définitions

*Définitions générales*

Par « *silicium organique »,* il faut comprendre un ou plusieurs composé(s) organo-silanol(s). Par « *glycotoxine »,* il faut comprendre l'ensemble des produits et sous-produits délétères résultant du processus de fixation de sucres réducteurs aux protéines.

Par « *complexe »* ou « complexe moléculaire », il faut comprendre une entité moléculaire formée par une association libre entre deux ou plusieurs entités moléculaires composantes (ioniques ou non chargées), ou les espèces chimiques correspondantes, la liaison entre les composants étant par ailleurs normalement plus faible que dans le cas d'une liaison covalente (Source: IUPAC, 1994, 66, 1077 - Glossary of terms used in physical organic chemistry - IUPAC - Recommendations 1994 - on page 1098).

Par « *liaison hydrogène »,* il faut comprendre une interaction attractive entre un atome d'hydrogène d'une molécule ou d'un fragment moléculaire X-H dans lequel X est plus électronégatif que H, et un atome ou un groupe d'atomes d'une autre molécule. Une liaison hydrogène typique peut être représentée par X-H•••Y-Z, où les trois points désignent la liaison et dans laquelle X-H représente le donneur de liaison hydrogène, l'accepteur étant une région riche en électrons tel qu'un doublet non liant sur Y. La preuve de la formation de liaison hydrogène peut être expérimentale ou théorique, ou une combinaison des deux (Henry M., Interference International Review of Science, 2015, et références citées).

Par « *en nomenclature systématique IUPAC »,* il faut comprendre une dénomination selon un système standardisé des composés chimiques basé sur l'application des recommandations les plus strictes établies par l'Union Internationale de Chimie Pure et Appliquée ("UICPA" en français, ou "IUPAC" en anglais pour "International Union of Pure and Applied Chemistry"), recommandations qui visent à s'assurer qu'un nom correspond à une et une seule structure moléculaire.

Par « *synergie d'action »,* il faut comprendre qu'il y ait effet synergique lorsque l'effet combiné de deux produits chimiques est supérieur à la somme des effets de chaque produit pris individuellement. Par exemple : 2 + 2 >> 4. [https://www.cchst.ca/oshanswers/chemicals/synergism.html].

Par « *dans le respect de ratio et conditions maîtrisés pour l'obtention d'un tel complexe moléculaire »,* il faut comprendre un rapport/ratio molaire dérivé organo-silanol de formule (I) / composé organique à motif imidazole de formule générale (II), établi après un criblage comparatif d'essais multiples visant à définir un ratio entre ces deux entités, susceptible de conférer au complexe selon l'invention un comportement biologique sur la peau qui puisse être le plus favorable possible, ainsi que l'identification de conditions opératoires qui permettent de maintenir simultanément une solubilisation des entités organo-silanol de formule (I) et composé organique à motif imidazole de formule générale (II) ainsi qu'une stabilisation de la première (et de ses groupes hydroxyles libres) par la seconde.

Par « *chlorhydrate »,* il faut comprendre le produit d'addition (sel) de l'acide chlorhydrique avec une base organique azotée. Il peut s'agir, par exemple, d'un chlorure, d'un bichlorure, etc.

*Définitions inhérentes au domaine cosmétique et dermocosmétique (non thérapeutique)*

Par *« protéine de la peau à demi-vie longue »,* il faut comprendre les protéines de la matrice extracellulaire dont les demi-vies atteignent plusieurs dizaines d'années, et qui constituent des cibles privilégiées du vieillissement moléculaire (Jaisson S. et al., Médecine/Sciences, 2017, vol.33, pp.176-182).

Par « *fibrose tissulaire associée à l'âge* », il faut comprendre la prédisposition des protéines de la matrice extra-cellulaire de la peau à développer, avec le vieillissement, un processus fibrotique tissulaire engendré par les nombreuses modifications (MPTNEs) ou "Modifications Post-Traductionnelles Non Enzymatiques" (glycosylation, réticulation, rigidité matricielle, etc.) auxquelles ces mêmes protéines sont sujettes (Selman M. et al., Ageing Res. Rev., 2021, vol.70, 101393).

## Description détaillée

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

## Exemples

Exemple 1 : il est mentionné ci-après deux illustrations de formulation de composition selon l'invention, à visée cosmétique ou dermocosmétique, toutes deux contenant un complexe selon l'invention précitée :

Formule A (crème)

Complexe selon l'invention [monométhylsilanetriol / 3-amino-N-[2-(1H-imidazol-5-yl)ethyl]propanamide (IUPAC)] : 5 %
Polyisobutène hydrogéné : 7 %
Myristate d'isobutyle : 3 %
Palmitate de cétyle : 7 %
Laurate sorbitan : 2 %
Polysorbate 20 : 2 %
Carbomer (copolymère d'acrylate/acrylamide & huile minérale) : 0,3 %
Phénoxyéthanol : 0,5 %
Eau : qsp 100%

Formule B (gel)

Complexe selon l'invention [diméthylsilanediol / acide(2S)-2-(3-aminopropanoylamino)-3-(1H-imidazol-5-yl) propanoïque (IUPAC)] : 5%
Carbomer (copolymère d'acrylate/acrylamide & huile minérale) :1,5 %
Benzoate de sodium : 0,2 %
Acide sorbique : 1 %
1,3-butanediol : 10 %
Glycérine : 5 %
Soude : 0,13 %
Phénoxyéthanol : 0,9 %
Eau : qsp 100 %

Exemple 2 : l'invention est illustrée ci-après, à titre purement indicatif par les tests suivants évoqués plus haut dans la description de l'invention (tests 1 à 4)

Test 1 : mise en évidence de la capacité du complexe selon l'invention à limiter la formation de la "BSA-glucosone"

L'évaluation de la formation de la "BSA-glucosone" par un complexe selon l'invention est effectuée *in tubo* dans les conditions ci-après définies, basée notamment sur des données de la littérature (Grzecgorczyk-Karolak I. et al., Molecules, 2016, vol.21, 739). Précisément, la protéine albumine bovine (BSA) (réf. Sigma A3912 - lot n°SLBJ0078V), à la concentration de 1 mg/ml, est incubée avec le D-glucosone (ou 2-keto-D-glucose), à savoir un composé carbonylé réactif ("RCC" ci-avant) produit par un réarrangement d'Amadori à partir d'un dérivé du glucose. Ainsi, 0,5 mM de D-glucosone (réf. Sigma 61793 - lot n° BCCK7499) est dissous dans de l'eau ultrapure, puis placé dans les conditions suivantes : pH 7,4, dans l'obscurité, à la température de 50°C et pendant 2 jours. Avant l'incubation, une solution des composés à tester dans de l'eau ultrapure est ajoutée au mélange ci-dessus. La formation de la "BSA-glucosone" est mesurée par fluorimétrie ($\lambda$ d'excitation : 340 nm ; $\lambda$ d'émission : 470 nm) en calculant le pourcentage de la formation de la "BSA-glucosone" à l'aide de l'équation suivante :

$$\% \text{ BSA—glucosone} = \frac{F_{traitement} - F_{traitement\ blanc}}{F_{contrôle} - F_{contrôle\ blanc}} \times 100$$

dans laquelle :

- "$F_{traitement}$" exprime l'intensité de fluorescence du composé à tester incubé avec du D-glucosone en présence de la BSA ;
- "$F_{traitement\ blanc}$" exprime l'intensité de fluorescence du composé à tester incubé avec du D-glucosone en absence de la BSA ;
- "$F_{contrôle}$" exprime l'intensité de fluorescence du D-glucosone en présence de la BSA ;
- "$F_{contrôle\ blanc}$" exprime l'intensité de fluorescence du D-glucosone en absence de la BSA.

Les résultats, répétés quatre fois, sont rassemblés dans le tableau 1 ci-après. Ils sont notamment exprimés en pourcentage de réduction sur la quantité produite de "BSA-glucosone". Ils ont été obtenus pour les différents configurations suivantes :

- cas 1, à savoir :
  - le "contrôle", soit la "BSA-glucosone" générée par la mise en contact de la glucosone sur la BSA et en absence de traitement ;
- cas 2, à savoir :
  - le composé monométhylsilanetriol [lots n° E17796 et 18521] objet de la formule (I) supra dans laquelle R = $CH_3$ (méthyle) et x = 1, testé seul;
- cas 3, à savoir :
  - le composé 3-amino-N-[2-(1H-imidazol-5-yl)ethyl]propanamide [lot n° E17797] objet de la formule (II) supra dans laquelle $R_1$ = H, $R_2$ est une chaîne alkyle de formule (III) dans laquelle $R_3$ est -H, $R_4$ est -$NHR_5$ avec $R_5$ étant -H, n est égal à 1, testé seul ;
- cas 4, à savoir :
  - le composé acide (2S)-2-(3-aminopropanoylamino)-3-(1H-imidazol-5-yl)propanoïque [réf. Sigma C9625- lot n° BCCB1338], objet de la formule (II) supra dans laquelle $R_1$ = $CO_2H$, $R_2$ est une chaîne alkyle de formule (III) dans laquelle $R_3$ est -H, $R_4$ est -$NHR_5$ avec $R_5$ étant -H, n est égal à 1, testé seul ;
- cas 5, à savoir :
  - le composé acide (4S)-4-amino-5-[2-(1H-imidazol-5-yl)ethylamino]-5-oxopentanoïque [lot n° 5116.24], objet de la formule (II) supra dans laquelle $R_1$ = H, $R_2$ est une chaîne alkyle de formule (III) dans laquelle $R_3$ est $NH_2$, $R_4$ est -$CO_2R_5$ avec $R_5$ étant -H, n est égal à 2, testé seul ;
- cas 6, à savoir :
  - le complexe selon l'invention, en solution aqueuse [lot n° E17767], associant (liaisons hydrogènes) les susnommés "composé monométhylsilanetriol" (cas 2) et "composé 3-amino-N-[2-(1H-imidazol-5-yl)ethyl]propanamide" (cas 3) dans le ratio molaire de 0,4 ;
- cas 7, à savoir :
  - le complexe selon l'invention, en solution aqueuse [lot n° E18562], associant (liaisons hydrogènes) les

susnommés "composé monométhylsilanetriol" (cas 2) et "composé acide (2S)-2-(3-aminopropanoylamino)-3-(1H-imidazol-5-yl)propanoïque" (cas 4) dans le ratio molaire de 0,36 ;

- cas 8, à savoir :

  • le complexe selon l'invention, en solution aqueuse [lot n° E18564], associant (liaisons hydrogènes) les susnommés "composé monométhylsilanetriol" (cas 2) et "composé acide (4S)-4-amino-5-[2-(1H-imidazol-5-yl)ethylamino]-5-oxopentanoïque" (cas 5) dans le ratio molaire de 0,38.

Tableau 1

| Composé (concentration) | quantité (%) de BSA-glucosone | diminution (%) sur production de BSA-glucosone |
|---|---|---|
| Cas 1 [contrôle] | 100 | N/A |
| Cas 2 (0,0625 %) | 98 | - 2 |
| Cas 3 (0,0625 %) | 99 | - 1 |
| Cas 6 [selon l'invention] (0,0625 %) | 87 | - 13 |
| Cas 2 (0,625 %) | 100 | 0 |
| Cas 4 (0,625 %) | 104 | +4 |
| Cas 7 [selon l'invention] (0,625 %) | 78 | - 22 |
| Cas 2 (0,625 %) | 100 | 0 |
| Cas 5 | 96 | - 4 |
| (0,625 %) | | |
| Cas 8 [selon l'invention] (0,625 %) | 71 | - 29 |

Les résultats soulignent, pour les complexes selon l'invention, contrairement à leurs deux éléments constitutifs pris isolément, une capacité à limiter la formation de la "BSA-glucosone" par un phénomène objectivé de synergie d'action, de surcroît pour le cas 6 dès une très basse concentration en actif de 0,0625 % (50 μM).

Test 2 : mise en évidence de la capacité du complexe selon l'invention à inhiber la sécrétion de l'enzyme protéolytique de type métalloprotéase matricielle "MMP-1" impliquée dans la protéolyse du collagène

Expérimentalement, le test est réalisé sur des cellules primaires de fibroblastes réticulaires et papillaires (abréviation "NHDF" pour "Normal Human Dermal Fibroblasts") obtenues suite à un isolement à partir d'une plastie mammaire (Alphenyx, lot 19-01-21001, patiente de 32 ans). Ces NHDF originellement maintenues à 37°C dans une atmosphère humide contenant 5% de $CO_2$, sont ensuite cultivées dans un milieu de culture complet "DMEM" (avec 4,5 g/l de glucose supplémenté avec 10% de sérum de veau fœtal (SVF) et 1% de glutamine. A J-3, les fibroblastes sont ensemencés en plaques de 6 puits à raison de $3.10^4$ cellules/$cm^2$. A J0, les cellules sont pré-traitées en présence de l'agent inhibiteur de référence, à savoir l'aminoguanidine (réf. Sigma : 396494 - lot n° MKCL5504) ou en présence des composés à tester. Les traitements sont réalisés dans du milieu "DMEM advanced" (DMEM avec 4,5 g/l de glucose et 2.5 mg/l d'acide ascorbique) supplémenté avec 1% de SVF pendant 7 jours. A J+7, l'agent de stress "RCC" (D-glucosone), à la concentration de 300 μM, est ensuite incubé pour une nouvelle période de 7 jours, en présence de l'agent inhibiteur de référence ou en présence des composés à tester. A J+14 (soit 14 jours de traitement au final dont 7 en présence de D-glucosone), les surnageants de culture sont récoltés afin de réaliser un dosage ELISA de la métalloprotéase matricielle « MMP-1 » (« kit Abcam, réf. ab215083 »). Les résultats obtenus sont ensuite soumis à une analyse statistique en triplicate. Les résultats, répétés trois fois, sont rassemblés dans le tableau 2 ci-après. Ils sont notamment exprimés en pourcentage d'inhibition sur la sécrétion de la "MMP-1". Ils ont été obtenus pour les différents configurations suivantes :
- cas 1, à savoir :

• le "contrôle" (sans ajout d'un complexe selon l'invention);

- cas 2, à savoir :

- l'agent inhibiteur de référence, avec la molécule "aminoguanidine" [réf. Sigma : 396495] ;

- cas 3, à savoir :

- le composé monométhylsilanetriol [lots n° E17855 et E18292] objet de la formule (I) supra dans laquelle R = $CH_3$ (méthyle) et x = 1, testé seul;

- cas 4, à savoir :

- le composé 3-amino-N-[2-(1H-imidazol-5-yl)ethyl]propanamide [lots n° E17797 et 182291] objet de la formule (II) supra dans laquelle $R_1$ = H, $R_2$ est une chaîne alkyle de formule (III) dans laquelle $R_3$ est -H, $R_4$ est -$NHR_5$ avec $R_5$ étant -H, n est égal à 1, testé seul;

- cas 5, à savoir :
• le complexe selon l'invention, en solution aqueuse [lot n° E17844 et E18290], associant (liaisons hydrogènes) les susnommés "composé monométhylsilanetriol" (cas 3) et "composé 3-amino-N-[2-(1H-imidazol-5-yl)ethyl]propanamide" (cas 4) dans le ratio molaire de 0,4.

Tableau 2

| Composé (concentration) | sécrétion (%) de MMP-1 | inhibition (%) sécrétion de MMP-1 |
|---|---|---|
| Cas 1 [contrôle] | 100 | N/A |
| Cas 2 [référence] (600 μM) | 63 | -37 |
| Cas 3 (0,1 %) | 77 | - 23 |
| Cas 4 (0,1 %) | 82 | - 18 |
| Cas 5 [selon l'invention] (0,1 %) | 70 | - 30 |

Les résultats soulignent, pour le complexe selon l'invention, une capacité largement supérieure, en comparaison de ses deux éléments constitutifs pris isolément, à inhiber la sécrétion de la MMP-1.

Test 3 : mise en évidence de la capacité du complexe selon l'invention à stimuler l'expression de la fibronectine

Expérimentalement, le test est réalisé sur de mêmes cellules primaires de fibroblastes réticulaires et papillaires que le test 2 ci-dessus, à savoir que lesdites cellules NHDF sont cultivées identiquement dans un milieu de culture complet "DMEM" (avec 4,5 g/l de glucose supplémenté avec 10% de sérum de veau fœtal (SVF) et 1% de glutamine). A J+1 après l'ensemencement, les cellules sont traitées pendant 3 jours en présence des composés à tester. Les cellules sont ensuite fixées afin de réaliser un immunomarquage en fluorescence de la fibronectine à l'aide d'un anticorps spécifique "anti-fibronectine (Abcam, réf. ab2413)" puis révélées avec un anticorps secondaire "Alexa Fluor 488® (Invitrogen, réf. A11008)". Le marquage est ensuite observé à l'aide d'un microscope à épifluorescence "Olympus BX60", au grossissement x20 pour chaque condition. Des photos sont prises avec une caméra DP72 Olympus à raison de trois photographies/condition. Il est enfin quantifié l'intensité moyenne de fluorescence du marquage à l'aide d'un logiciel d'analyse d'images (logiciel "ImageJ®"). Les résultats de chaque condition ont été comparés à la condition témoin. Les résultats, présentés en % moyen (en triplicate, n=3), sont rassemblés dans le tableau 3 ci-après. Ils sont notamment exprimés en pourcentage de stimulation sur l'expression de la fibronectine. Ils ont été obtenus pour les différents configurations suivantes:

- cas 1, à savoir :
  - le "témoin" (sans ajout d'un ingrédient actif) ;
- cas 2, à savoir :
  - le composé monométhylsilanetriol [lot n° E18443], objet de la formule (I) supra dans laquelle R = $CH_3$ (méthyle) et x = 1, testé seul;
- cas 3, à savoir :

- le composé 3-amino-N-[2-(1H-imidazol-5-yl)ethyl]propanamide [lot n° E18398] objet de la formule (II) supra dans laquelle $R_1$ = H, $R_2$ est une chaîne alkyle de formule (III) dans laquelle $R_3$ est -H, $R_4$ est -$NHR_5$ avec $R_5$ étant -H, n est égal à 1, testé seul;

- cas 4, à savoir :

    - le complexe selon l'invention, en solution aqueuse [lot n° E18442], associant (liaisons hydrogènes) les susnommés "composé monométhylsilanetriol" (cas 2) et "composé 3-amino-N-[2-(1H-imidazol-5-yl)ethyl]propanamide" (cas 3) dans le ratio molaire de 0,4.

Tableau 3

| Composé (concentration) | expression (%) fibronectine | majoration (%) expression fibronectine |
|---|---|---|
| Cas 1 [témoin] | 100 | N/A |
| Cas 2 (0,1 %) | 104 | +4 |
| Cas 3 (0,1 %) | 121 | + 21 |
| Cas 4 [selon l'invention] (0,1 %) | 138 | + 38 |

Les résultats soulignent, pour le complexe selon l'invention, en comparaison de ses deux éléments constitutifs pris isolément, une capacité très largement supérieure, de surcroît même par un phénomène objectivé de synergie d'action, à stimuler l'expression de la fibronectine.

Test 4 : procédé de préparation d'un complexe de silicium organique selon l'invention (à savoir le susnommé complexe tel que ledit organo-silanol de formule générale (I) et ledit composé organique à motif imidazole de formule générale (II) étant respectivement le monométhylsilanetriol et le composé 3-amino-N-[2-(1H-imidazol-5-yl)ethyl]propanamide)

20 g (78 mmoles) de composé 3-amino-N-[2-(1H-imidazol-5-yl)ethyl]propanamide), sous sa forme chlorhydrate, sont dissous dans 720 g d'eau déminéralisée. Sous le contrôle du pH (entre 3 et 8) et un ajustement avec une solution d'HCl, une solution (7,25 g) commerciale de méthylsiliconate de sodium (32 mmoles), préalablement diluée dans environ 96 ml d'eau, est ajoutée progressivement à la solution précédente sous agitation, à température ambiante. La masse de solution est finalement ajustée à 1kg par addition d'eau déminéralisée. La solution résultante de complexe (ratio molaire de 0,4 entre le monométhylsilanetriol et le composé 3-amino-N-[2-(1H-imidazol-5-yl)ethyl]propanamide) est finalement ajustée à un pH de 4,9 à l'aide de quelques gouttes d'HCl. On obtient une solution limpide, incolore et inodore.

## Revendications

**1.** Complexe de silicium organique formé entre :

a) un organo-silanol de formule générale (I) suivante :

$$(R)_x\text{-}Si(OH)_{4-x} \qquad (I)$$

dans laquelle :

- le radical R est un groupement alkyle en $C_1$-$C_4$, linéaire ou ramifié, de préférence en $C_1$-$C_2$, éventuellement substitué par au moins un groupement hydroxyle, de préférence R est un groupement méthyle, et
- x est égal à 1 ou 2 ; et

b) au moins un composé organique à motif imidazole de formule générale (II) suivante, ou l'un de ses sels cosmétiquement ou dermocosmétiquement acceptables :

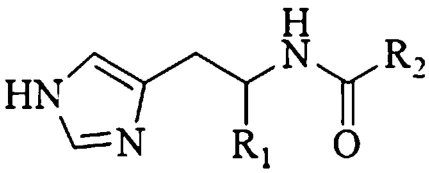

(II)

dans laquelle :

- $R_1$ est -H, $-CO_2H$ ou $-CH_2OH$,
- $R_2$ est :

i) soit une chaîne alkyle de formule (III) suivante :

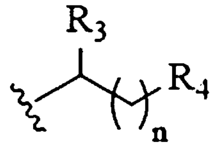

(III)

dans laquelle :

- $R_3$ est -H ou $-NH_2$ ;
- $R_4$ est $-CO_2R_5$ ou $-NHR_5$, avec $R_5$ étant -H ou $-COCH_3$;
- n est égal à 1 ou 2 ;

ii) soit un hétérocycle choisi parmi:

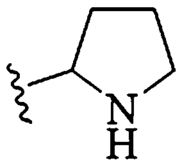

et

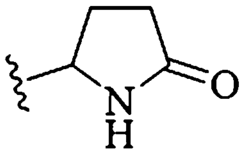

ledit complexe étant formé via l'établissement d'au moins une liaison faible, de préférence d'au moins une liaison hydrogène, entre au moins un groupement hydroxyle dudit organo-silanol de formule générale (I) et ledit au moins un composé organique à motif imidazole de formule générale (II), de préférence au niveau d'au moins un des atomes d'azote et d'oxygène dudit au moins un composé organique à motif imidazole de formule générale (II); le ratio molaire entre ledit organo-silanol de formule générale (I) et ledit au moins un composé organique à motif imidazole de formule générale (II) étant compris entre environ 0,1 et environ 1.

**2.** Complexe selon la revendication 1, dans lequel ledit organo-silanol de formule générale (I) est le monométhylsilanetriol ou le diméthylsilanediol, de préférence le monométhylsilanetriol.

**3.** Complexe selon l'une des revendications précédentes, dans lequel ledit au moins un composé organique à motif imidazole de formule générale (II) est tel que :

- $R_1$ est -H ou $-CO_2H$ ; et
- $R_2$ est une chaîne alkyle de formule (III) dans laquelle :

◦ $R_3$ est -H,
◦ $R_4$ est $-NHR_5$ avec $R_5$ étant -H ou $-COCH_3$,

◦ n est égal à 1.

**4.** Complexe selon l'une des revendications précédentes, dans lequel ledit au moins un composé organique à motif imidazole de formule générale (II) est le composé 3-amino-N-[2-(1H-imidazol-5-yl)ethyl]propanamide, le composé 3-acetamido-N-[2-(1H-imidazol-5-yl)ethyl]propanamide, le composé acide (2S)-2-(3-aminopropanoylamino)-3-(1H-imidazol-5-yl)propanoïque, et/ou le composé acide (4S)-4-amino-5-[2-(1*H*-imidazol-5-yl)ethylamino]-5-oxopentanoïque ; de préférence ledit au moins un composé organique à motif imidazole de formule générale (II) étant le composé 3-amino-N-[2-(1H-imidazol-5-yl)ethyl]propanamide.

**5.** Complexe selon l'une des revendications précédentes, dans lequel ledit sel cosmétiquement ou dermocosmétiquement acceptable dudit au moins un composé organique à motif imidazole de formule générale (II) est un chlorhydrate.

**6.** Complexe selon l'une des revendications précédentes, dans lequel ledit organo-silanol de formule générale (I) et ledit au moins un composé organique à motif imidazole de formule générale (II) sont respectivement le monométhylsilanetriol et le 3-amino-N-[2-(1H-imidazol-5-yl)ethyl]propanamide.

**7.** Complexe selon l'une des revendications précédentes, dans lequel le ratio molaire entre ledit organo-silanol de formule générale (I) et ledit au moins un composé organique à motif imidazole de formule générale (II) est compris entre environ 0,15 et environ 0,8, avantageusement entre environ 0,2 et environ 0,6.

**8.** Composition cosmétique ou dermocosmétique, **caractérisée en ce que** ladite composition comprend, en association avec au moins un excipient cosmétiquement ou dermocosmétiquement acceptable, au moins un complexe selon l'une des revendications précédentes, de manière préférée ladite composition étant sous une forme adaptée à une administration par voie topique cutanée et ledit au moins un excipient étant en outre physiologiquement acceptable avec la peau.

**9.** Composition cosmétique ou dermocosmétique selon la revendication précédente, **caractérisée en ce que** ladite composition, comprend en outre au moins un ingrédient actif additionnel choisi parmi les agents dépigmentants, les agents photoprotecteurs, les agents stimulant la fonction barrière, les agents hydratants ou humectants, les agents desquamants, les agents apaisants, les agents exfoliants, les agents stimulant la prolifération cellulaires tels les fibroblastes et les kératinocytes, les agents déglycosylants, les agents stimulant la synthèse de collagène ou d'élastine ou prévenant leur dégradation, les agents stimulant la synthèse de glycosaminoglycanes ou de protéoglycanes ou prévenant leur dégradation, les agents antioxydants ou anti-radicalaires ou anti-pollution, les agents stimulant la lipolyse, les agents drainants ou détoxifiants, les agents anti-inflammatoires, les agents accélérateurs de pénétration et/ou anti-irritants, les agents astringents, les agents agissant sur la microcirculation, et leurs mélanges.

**10.** Utilisation cosmétique ou dermocosmétique non-thérapeutique d'un complexe selon l'une des revendications 1 à 7 ou d'une composition selon la revendication 8 ou 9, pour maintenir et/ou augmenter l'expression des protéines de la matrice extracellulaire de la peau saine, de préférence ladite utilisation étant une utilisation par voie topique cutanée.

**11.** Utilisation selon la revendication 10, dans laquelle les protéines de la matrice extracellulaire de la peau saine sont la fibronectine.

**12.** Utilisation cosmétique ou dermocosmétique non-thérapeutique d'un complexe selon l'une des revendications 1 à 7 ou d'une composition selon la revendication 8 ou 9 :

a) pour maintenir et/ou augmenter les propriétés biomécaniques de la peau saine, préférentiellement la fermeté et/ou l'élasticité de la peau saine, et/ou
b) pour la prévention et/ou la correction des signes de vieillissement de la peau saine.

**13.** Utilisation cosmétique ou dermocosmétique non-thérapeutique d'un complexe selon l'une des revendications 1 à 7 ou d'une composition selon la revendication 8 ou 9, pour restaurer le réseau vasculaire cutané altéré avec l'âge de la peau saine.

**14.** Utilisation d'au moins un composé organique à motif imidazole de formule générale (II) suivante, ou l'un de ses sels cosmétiquement ou dermocosmétiquement acceptables :

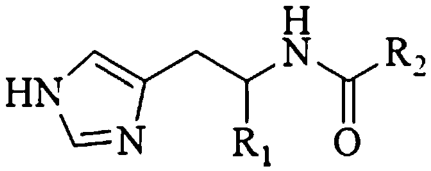

(II)

dans laquelle :

- $R_1$ est -H, $-CO_2H$ ou $-CH_2OH$,
- $R_2$ est :

i) soit une chaîne alkyle de formule (III) suivante :

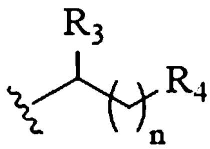

(III)

dans laquelle :

- $R_3$ est -H ou $-NH_2$ ;
- $R_4$ est $-CO_2R_5$ ou $-NHR_5$, avec $R_5$ étant -H ou $-COCH_3$ ;
- n est égal à 1 ou 2 ;

ii) soit un hétérocycle choisi parmi:

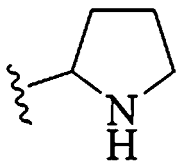

et

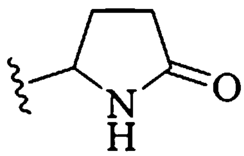

pour stabiliser un organo-silanol de formule générale (I) suivante :

$$(R)_x\text{-Si}(OH)_{4-x} \qquad (I)$$

dans laquelle :

- le radical R est un groupement alkyle en $C_1$-$C_4$, linéaire ou ramifié, de préférence en $C_1$-$C_2$, éventuellement substitué par au moins un groupement hydroxyle, de préférence R est un groupement méthyle, et
- x est égal à 1 ou 2.

Europäisches Patentamt
European Patent Office
Office européen des brevets

# RAPPORT DE RECHERCHE EUROPEENNE

Numéro de la demande
EP 25 21 8621

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2015/004113 A1 (RITTER HELMUT [DE] ET AL) 1 janvier 2015 (2015-01-01)<br>* exemple 10 page 17 *<br>----- | 1-14 | INV.<br>C07F7/18<br>A61K8/58<br>A61Q19/00<br>A61Q19/08<br>C07D247/02 |
| | | | DOMAINES TECHNIQUES RECHERCHES (IPC) |
| | | | C07F<br>C07D<br>A61Q<br>A61K |

Le présent rapport a été établi pour toutes les revendications

1

| Lieu de la recherche | Date d'achèvement de la recherche | Examinateur |
|---|---|---|
| La Haye | 10 avril 2026 | Bourghida, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 25 21 8621

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-04-2026

| Document brevet cité au rapport de recherche | Date de publication | Membre(s) de la famille de brevet(s) | Date de publication |
|---|---|---|---|
| US 2015004113 A1 | 01-01-2015 | CN 104277227 A | 14-01-2015 |
| | | DE 102013106906 A1 | 08-01-2015 |
| | | EP 2821429 A1 | 07-01-2015 |
| | | US 2015004113 A1 | 01-01-2015 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20150004113 A
- FR 3038898
- EP 2172186 A
- EP 4153603 A


### Littérature non-brevet citée dans la description


- Bulletin de l'OMS. *Vieillissement et santé*, 01 October 2024
- **BOISMAL F. et al.** *Médecine/Sciences*, 2020, vol. 36, 1163-72
- **JAISSON S. et al.** *Médecine/Sciences*, 2017, vol. 33, 176-182
- **JAISSON S. et al.** *Adv. Clin. Chem.*, 2018, vol. 84, 1-38
- **GILLERY P. et al.** *Clin. Chem. Lab. Med.*, 2013, vol. 92, 228-238
- **KENT M.J.C.** *Biochem. J.*, 1985, vol. 225, 745-752
- **DEFAYE J. et al.** *L'Actualité Chimique*, 2000, 24-27
- **SCHLIENGER J.L.** *Médecine des Maladies Métaboliques*, 2022, vol. 6, 567-572
- **TESSIER F.J.** *Pathologie Biologie*, 2010, vol. 58, 214-219
- **GILLERY P.** *J. Med. Biochem.*, 2011, vol. 30, 201-206
- **GILLERY P. et al.** *Clin. Chem. Lab. Med.*, 2014, vol. 52, 33-38
- **MONTEIRO-ALFREDO T. et al.** *Diabetology*, 2022, vol. 3, 596-605
- **ODETTI P. et al.** *Mediterr. J. Nutr. Metab.*, 2008, vol. 1, 63-67
- **KUZAN A.** *Biomed. Rep.*, 2021, vol. 14, 1-8
- **BUSZKA A.** *Aesthetic Cosmetology and Medicine*, 2023, vol. 12, 23-278
- **GKOGKOLOU P. et al.** *Dermato-Endocrinology*, 2012, vol. 4, 259-270
- **WANG L. et al.** *Exp. Derm.*, 2024, vol. 33, e15065
- **J. URIBARRI et al.** *J. Am. Diet. Asso.*, 2010, vol. 110, 911-916
- **GILLERY P.** *J. Soc. Biol.*, 2001, vol. 195, 387-390
- **ZHENG W. et al.** *Nutrients*, 2022, vol. 14, 1-19
- **UCEDA A.B. et al.** *Biophys. Rev.*, 2024, vol. 16, 189-218
- **VELICHKOVA S. et al.** *Planta Med.*, 2021, vol. 87, 780-802
- **SONG Q. et al.** *Biomed. Pharmacotherap.*, 2021, vol. 140, 111750
- **JACOB M.P.** *Médecine/Sciences*, 2006, vol. 22, 273-278
- **HENRY M.** *Interference International Review of Science*, 2015
- **SELMAN M. et al.** *Ageing Res. Rev.*, 2021, vol. 70, 101393
- **GRZECGORCZYK-KAROLAK I. et al.** *Molecules*, 2016, vol. 21, 739